# EUROPEAN PATENT SPECIFICATION

(11) **EP 1 987 804 B1**
(45) Date of publication and mention of the grant of the patent: **29.12.2010**
(21) Application number: 07008902.4
(22) Date of filing: 02.05.2007
(51) Int. Cl.: A61F 2/44, A61K 31/728, A61K 38/36, A61K 38/48

(54) **Implantable system for an intervertebral disc and intervertebral disc implant**
Implantierbares System für eine Bandscheibe und Bandscheibenimplantat
Système implantable pour un disque intervertébral et implant de disque intervertébral

(43) Date of publication of application: 05.11.2008
(73) Proprietor: Klinikum Mannheim GmbH, 68167 Mannheim (DE)
(72) Inventor: Hegewald, Aldemar, 68167 Mannheim (DE)
(74) Representative: Müller-Boré & Partner Patentanwälte

(56) References cited:
- WO-A-2004/067704
- WO-A-2005/060987
- WO-A-2006/050269
- US-A1- 2005 196 387

## Description

The present invention relates to an implantable system comprising a biologically acceptable bag comprising a cell carrier medium and a multi-component system, the use of such an implantable system for implanting into an intervertebral disc for the treatment of an annulus fibrosus and/or nucleus pulposus defect and/or for preventing an annulus fibrosus and/or nucleus pulposus defect, and an intervertebral disc implant.

A disc herniation is a medical condition affecting the intervertebral disc linking two adjacent vertebral bodies in the spine. The intervertebral disc consists of a gelatinous nucleus pulposus which is surrounded by a laminated fibrous ring, namely the annulus fibrosus. The nucleus pulposus basically contains collagen fibrils, hyaluronic acid and water-binding glycosaminoglycans. However, the water content of the nucleus pulposus decreases with age which reduces its ability to withstand stress. As a consequence, in combination with general wear and tear on the intervertebral disc over time, repetitive movements or stress on the disc that occurs while twisting and lifting, the nucleus pulposus may flatten and bulge out radially. This leads to a disc protrusion which is a condition in which the outermost layers of the annulus fibrosus are still intact, but can bulge when the disc is under pressure. In a further development, the annulus fibrosus of the intervertebral disc is radially ruptured and parts of the nucleus pulposus prolapse through the ruptured annulus fibrosus. Said condition is generally referred to as disc herniation and very often leads to compression of the spinal canal and pressure on the nerve roots that pass through the spinal canal. This usually causes a strong and progressive pain that emanates from the compromized segment of the spine.

The disc herniation condition is first treated conservatively with physical therapy and anti-inflammatory drugs or oral steroids. With persisting pain or the incidence of neurologic deficits a surgical intervention is recommended. The classical surgical treatment for herniated disc is discectomy. Due to accelerated intervertebral disc degeneration many patients develop devastating back pain in the following years, defined as intervertebral disc disease. These patients receive spinal fusion or disc arthroplasty procedures known to be invasive, having high complication rates and which are considered to show significant long-term limitations.

In discectomy, protruding annulus fibrosus and nucleus pulposus material as well as nucleus pulposus within the disc are removed. The resulting reduction in the volume of disc material leads to decreased pressure on the compressed nerve roots and/or the spinal cord, thus generally leading to a pain relief for the patient.

However, both the conservative therapy as well as the conventional surgical intervention has significant drawbacks. In particular, the affected spine segment very often shows accelerated degenerative changes due to a biomechanical instability and reduced disc height. Said degenerative changes of the affected spine segment may cause lasting back pain for the patient. In addition, because of the defect of the annulus fibrosus, re-herniation of the disc may occur. Because of such a re-herniation, 7 to 21 % of the patients having received a discectomy surgery need to be operated again.

In order to overcome the above drawbacks, regenerative therapy approaches for the intervertebral disc have been proposed. Such regenerative therapy approaches in degenerative spinal surgery are intended to generate healthy disc tissue or functional surrogate tissue in order to avoid or to reverse painful degeneration processes.

Another alternative are artificial nucleus pulposus prostheses. These may be implanted in the form of pads or spirals or as injectable components which obtain their final strength after introduction into the disc. Artificial nucleus pulposus prostheses are generally based on hydrogel implants or *in situ* curable polyurethane implants. However, such prostheses are generally permanent implants which require observation of long term biological responses throughout the life of the prosthesis. Moreover, migration of the implant has been observed, the integration of the implant into the surrounding tissue is very often not sufficient, and a loss of the biomechanical properties may occur with time. Furthermore, in case a hydrogel or polyurethane based implant is used, re-herniation is very likely to occur through the still damaged annulus fibrosus. For intervertebral disc disease, total disc replacement is used now widely in the clinical routine as an alternative to spinal fusion. However, up to now trials have failed to show its superiority toward spinal fusion.

Different regenerative treatment strategies have been developed. For example, in the autologous disc cell transplantation, nucleus pulposus tissue is extracted from the patient when performing a conventional discectomy. Nucleus pulposus cells are isolated and expanded *in vitro,* and then percutaneously re-implanted into the patient after about twelve weeks. Said method enables repair of the nucleus pulposus by new matrix synthesis of the intervertebral disc. However, said method has the drawback of providing an insufficient initial biomechanical stability as well as an unregular distribution of the re-implanted nucleus pulposus cells. Further, US 2001/0020476 A1 describes a method of reforming intervertebral disc tissue comprising the steps of evacuating intervertebral disc tissue from a degenerated nucleus pulposus, preparing a hybrid material by combining the intervertebral disc cells with a biodegradable substrate, and implanting the hybrid material in the evacuated nucleus pulposus space. Also DE 10 2004 043 449 A1 describes a method for the *in vitro* preparation of an intervertebral disc chondrocyte based implant from degenerated intervertebral disc tissue of a patient and its application as transplantation material for the treatment of a defect intervertebral disc. It has also been reported to use autologous mesenchymal stem cells for the regeneration of a degenerated intervertebral disc. For example, US 2006/0153812 A1 describes a medium for stem cell and the use of said medium in a method for regeneration of an intervertebral disc. However, all of the above regenerative methods suffer from the drawback that re-herniation is very likely to occur through the still damaged annulus fibrosus. Further, adequate nutrition might pose a problem in strongly degenerated cartilage endplates of the adjacent vertebral bodies.

In order to prevent re-herniation, methods of sealing the annulus fibrosus have been suggested. For example, US-patent 6,428,576 describes a method of sealing a defect in the annulus fibrosus by applying a curable biocompatible material to the defect, and curing that material *in situ* into a cross-linked visco-elastic polymer adhering to remaining annulus fibrosus and thereby closing said defect. However, such annulus fibrosus sealing techniques are problematic in view of a possible migration of the used synthetic materials.

WO2004/067704 describes porous fibrin matrices formed from plasma proteins comprising fibrinogen, thrombin and Factor XIII and further comprising hyaluronic acid. Said matrices may be used clinically, per se or as cell-bearing implants.

Accordingly, the technical problem underlying the present invention is to provide a system to be implanted in an intervertebral disc, wherein said implantable system should be highly biocompatible, should prevent the occurrence of re-herniation, should provide a high initial biomechanical stability, and should enable adequate regeneration of new intervertebral disc tissue as well as enhance nutrient supply.

The solution to the above technical problem is achieved by providing the embodiments characterized in the claims.

In particular, the present invention relates to an implantable system for an implant into an intervertebral disc, comprising a biologically acceptable bag comprising a cell carrier medium, and a multi-component system comprising:
component (a) containing fibrinogen;
component (b) containing thrombin; and
component (c) containing hyaluronic acid.

Surprisingly, the system according to the present invention when being implanted into a patient provides excellent implant characteristics such as a high biologically acceptance, a high durability and a high mechanical stability. Further, the implantable system according to the present invention when being implanted provides the basis for the regeneration of damaged intervertebral disc tissue.

As used herein, the term "patient" means a subject suffering from an annulus fibrosus and/or nucleus pulposus defect and includes mammals, particularly horses and human beings. For example, said annulus fibrosus and/or nucleus pulposus defect may be associated with a spinal disc herniation or may be the consequence of a spinal disc herniation or may be an intervertebral disc disease.

According to the present invention, the biologically acceptable bag may be made of any suited biologically acceptable material. The form, size, thickness and fiber direction of the bag may be selected in a manner suitable in view of the particular biomechanical and physiological demands. In one preferred embodiment of the present invention, the biologically acceptable bag is biodegradable and/or bioresorbable which is advantageous from the viewpoint of a rapid integration of the implanted bag into the surrounding tissues. In another preferred embodiment of the present invention, the biologically acceptable bag is not or only hardly biodegradable and/or bioresorbable. This is advantageous from the viewpoint of the prevention of a re-hernation, since the bag being implanted into the intervertebral disc keeps the newly formed nucleus pulposus permanently within the annulus fibrosus. Further, it is preferable that the bag is made of a soft and/or flexible material. This is favorable in view of the adaption of the bag into the intervertebral disc space in which the bag is to be implanted and offers the opportunity of minimally invasive insertion of the bag.

Further, the bag may be sealable or sealed. In particular, the bag may have an opening which is sealed or sealable, i.e. the opening may be sealed for example before or after the bag has been introduced into the intervertebral disc. Preferably, the bag has a neck-like opening which is advantageous from the viewpoint of conveniently filling the multi-component system into the bag e.g. by means of a needle of a syringe. The bag may be sealed before or after being filled with the multi-component system. When the bag is sealed, the bag may be filled for example by puncturing the bag with the needle of a syringe and injecting the multi-component system into the bag through the puncture. In another preferred embodiment of the present invention, the biologically acceptable bag is initially open and is sealed at a later stage, e.g. after being implanted into the intervertebral disc or after being filled with the multi-component system.

The bag may be sealed by any means which are suitable for such purpose in a biologically acceptable manner. For example, the bag may be sealed with a suture e.g. in the form of a pursestring suture, and/or by means of suitable adhesives. Suitable adhesives are for example fibrin-based adhesives such as the commercially available TachoSil^{®}.

Further, the biologically acceptable bag comprises a cell carrier medium which may be any suitable cell carrier medium. In particular, the cell carrier medium is intended to act as a three-dimensional framework which allows cells to adhere to, to differentiate and to synthesize and form a matrix. Further, the cell carrier medium preferably supports the formation of complex tissue structures. In one preferred embodiment of the present invention, the material of which the bag is made corresponds to the cell carrier medium. It is further preferred that the biologically acceptable bag is permeable for nutrients and biological degradation products. This is desirable from the viewpoint of allowing the cells within the bag to grow in order to form complex tissue structures. In a preferred embodiment of the present invention, the cell carrier medium is a bioresorbable polymer fleece comprising a material selected from the group consisting of collagens, hyaluronic acid, glycosaminoglycane, polylactic acid, polyglycolic acid, copolymers of polylactic acid and polyglycolic acid, and combinations thereof. Suitable bioresorbable polymer fleeces are commercially available, e.g. under the trademark Ethisorb^{®} distributed by Ethicon Company being made of polyglactin 910 (VICRYL^{®}) or poly-p-dioxanone (PDS^{®}). The cell carrier medium may further contain e.g. inorganic compounds selected from the group consisting of hydroxy apatite, calcium phosphate, metals, calcium sulfate, and combinations thereof, and/or organic compounds selected from the group consisting of demineralized bone particles and/or matrix, small intestine submucosa powder, and combinations thereof.

In a preferred embodiment of the present invention, the components (a) to (c) of the multi-component system as defined above are each present in solution, and at least component (a) is spacially separated from component (b).

The term "fibrinogen" includes not only fibrinogen *per se*, but also any clot-forming substance, such as clot-forming derivatives of fibrinogen, for example "fibrin1 ".

The fibrinogen component (a) of the multi-component system as defined above may further comprise one or more of extracellular matrix proteins, for example fibronectin, cellular associated proteins, other plasma derived proteins, for example blood clotting factor XIII (FXIII) and proteases, and protease inhibitors, and mixtures thereof. The fibrinogen component according to the present invention may also include any additive which is comprised in the state of the art for scientific and/or commercially available fibrinogen compositions, for example commercially available fibrinogen solutions.

The amount of fibrinogen in component (a) of the multi-component system may be within any suitable range. In a preferred embodiment of the present invention, the amount of fibrinogen in component (a) ranges from about 10 to about 200 mg/ml, more preferable from about 30 to about 150 mg/ml, and still more preferable from about 75 to about 115 mg/ml.

Further, the term "thrombin" includes not only thrombin *per se*, but also any gelation-inducing or clotting-inducing agent for component (a), for example a physiologically acceptable alkaline buffer system, or a precursor of thrombin.

The thrombin component (b) of the multi-component system according to the present invention may further comprise additional compounds known in the art. There is no specific limitation in respect to the used amount of thrombin. In a preferred embodiment of the present invention, the amount of thrombin in said thrombin component (b) is at least about 1 IU/ml in the final clotted composition, more preferably at least about 30 IU/ml.

Moreover, the term "hyaluronic acid" refers to any polymer of disaccharides composed of D-glucuronic acid and N-acetyl-D-glucosamine, linked together via alternating β-1,4 and β-1,3 glycosidic bonds. Polymers of hyaluronic acid generally have a size within the range of from about 5,000 to about 20,000,000 Da. Suitable hyaluronic acids are commercially available, e.g. under the tradenames Ostenil^{®} and Hylartil^{®}.

There is no specific limitation in respect to the used hyaluronic acid amount. In a preferred embodiment of the present invention, the amount of hyaluronic acid in said hyaluronic acid component (c) ranges from about 0.01 to about 5 mg/ml, more preferably from about 0.1 to about 3 mg/ml.

In another preferred embodiment of the present invention, the multi-component system further comprises autologous serum. Within the scope of the present application, the term "autologous serum" refers to serum which is derived from the patient in which the implantable system is to be implanted. There is no specific limitation in respect to the used amount of autologous serum and the person skilled in the art will know in which amount the autologous serum is to be included into the multi-component system. The autologous serum may be used as the medium in which the other components are dissolved and/or suspended. Further, the autologous serum may act as a source of nutrients which is advantageous from the viewpoint of a regeneration of the damaged nucleus pulposus and/or annulus fibrosus. Moreover, autologous serum may contain bioactive factors which are suitable to enforce matrix production of nucleus pulposus cells.

According to the present invention, the nucleus pulposus of the damaged spinal disc is preferably at least partially regenerated. This is achieved by the growth of new tissues within the bag of the implantable system. Said new tissue is derived from suitable cells within the bag. Chemotactic molecules for nucleus pulposus cells or precursor cells like mesenchymal stem cells may further be included in the multi-component system in order to further facilitate *in situ* cell recruitment. Suitable cells for the growth of new nucleus pulposus material may be derived from the surrounding tissues such as the remainder of the damaged nucleus pulposus, wherein the cells diffuse through the permeable bag into the bag. In combination with the cell carrier medium and/or the multi-component system, said cells form the basis for the growth of new nucleus pulposus material.

In another preferred embodiment of the present invention, it is possible to add such cells together with the multi-component system. In particular, the multi-component system preferably further comprises cells selected from the group consisting of autologous stem or precursor cells, allogenic stem or precursor cells, autologous or allogenic periost cells, autologous or allogenic chondrocytes, autologous or allogenic nucleus pulposus cells, autologous or allogenic annulus fibrosus cells and combinations thereof. Methods for obtaining autologous stem cells, precursor cells, chondrocytes, annulus fibrosus cells and nucleus pulposus cells from a patient are known in the state in the art. Further, also methods for the extraction of allogenic stem and/or precursor cells from a donor different than the patient are known in the state of the art. By including such cells into the multi-component system, it is possible to regenerate at least partially the damaged nucleus pulposus after implantation. It is particularly preferable to use autologous mesenchymal stem cells (MSC), since said stem cells are exceptionally capable to grow in biopolymers comprising hyaluronic acid and to build up an extracellular matrix. It is also particularly preferable to use autologous nucleus pulposus cells, since autologous nucleus pulposus cells are suitable to form the basis for the regeneration of the nucleus pulposus in the damaged spinal disc.

For the regeneration of the nucleus pulposus in the damaged spinal disc, it is advantageous that the multi-component system further comprises ingredients selected from the group consisting of annulus fibrosus stimulating factors, annulus fibrosus specific matrix components, anti-inflammatory substances, nucleus pulposus stimulating factors, nucleus pulposus specific matrix components, and combinations thereof. Preferred examples for nucleus pulposus specific matrix components are collagens, in particular collagen type II.

The above multi-component system may further include any other component suitable for e.g. augmenting, strengthening, supporting, repairing, rebuilding or healing a intervertebral disc, such as growth factors, chemotherapeutic or pharmacological agents, biologically active agents, hardening and/or adhesive compounds and mineral additives. These compounds may be contained in any of the components (a) to (c) of the multi-component system according to the present invention or may be comprised as extra components.

The present invention further relates to an intervertebral disc implant comprising a biologically acceptable bag comprising a cell carrier medium, the bag comprising a composition comprising
component (a) containing fibrin;
component (b) containing thrombin; and
component (c) containing hyaluronic acid.

The intervertebral disc implant according to the present invention is obtainable by implanting the above implantable system into an intervertebral disc of a patient suffering from an annulus fibrosus and/or nucleus pulposus defect. For example, said annulus fibrosus and/or nucleus pulposus defect may be associated with a spinal disc herniation or may be the consequence of a spinal disc herniation or may be an intervertebral disc disease.

In particular, the intervertebral disc implant is prepared from the implantable system as defined above, for example by introducing the bag comprising a cell carrier medium into an intervertebral disc space, mixing components (a) to (c) of said multi-component system together and/or homogenizing said components, filling components (a) to (c) of the multi-component system into the sealable bag, and sealing the bag. The preparation of the intervertebral disc implant can be carried out at any suitable temperature, such as in the range from about 18 to about 37°C, for example at 25°C.

According to the present invention, the bag of the intervertebral disc implant is preferably sealed. The bag may be sealed by any means which are suitable for such purpose in a biologically acceptable manner. For example, the bag may be sealed with a suture e.g. in the form of a pursestring suture, and/or by means of suitable adhesives. Suitable adhesives are for example fibrin-based adhesives such as the commercially available TachoSil^{®}. In a preferred embodiment of the present invention, the bag is sealed by a pursestring suture.

The biologically acceptable bag comprising a cell carrier medium and components (b) and (c) of the intervertebral disc implant according to the present invention are the same as defined for the implantable system characterized above.

The term "fibrin" does not only refer to fully coagulated fibrinogen but further includes any mixture of fibrin and fibrinogen which may occur during formation of fibrin from fibrinogen using thrombin and, thus, includes any ratio of fibrinogen/fibrin and any grade of gelation and/or clotting conceivable as long as it has no negative impact on the final composition filled into the bag of the intervertebral disc implant. The fibrin component (a) of the intervertebral disc implant of the present invention further includes fibrin with only a small amount of fibrinogen or without any fibrinogen left in said fibrin. Moreover, the term "fibrin" further includes any partly or fully gelled or clotted form of component (a) as defined above.

The amount of fibrin in component (a) of the composition included in the intervertebral disc implant may be within any suitable range. In a preferred embodiment of the present invention, the amount of fibrin in component (a) ranges from about 10 to about 200 mg/ml, more preferable from about 30 to about 150 mg/ml, and still more preferable from about 75 to about 115 mg/ml in the final clotted composition.

There is no specific limitation in respect to the amount of thrombin in the composition. In a preferred embodiment of the present invention, the amount of thrombin in said thrombin component (b) is at least about 1 IU/ml in the final clotted composition, more preferably at least about 30 IU/ml.

There is no specific limitation in respect to the hyaluronic acid amount in the composition included in the bag of the intervertebral disc implant. In a preferred embodiment of the present invention, the amount of hyaluronic acid in said hyaluronic acid component (c) ranges from about 0.01 to about 5 mg/ml, more preferably from about 0.1 to about 3 mg/ml.

According to the present invention, the composition as defined above is in a gelled or clotted state and has a viscosity suitable for injecting into the bag of the intervertebral disc implant being introduced into the disc space of an intervertebral disc, and may be applied in a pre-clotted liquid, gelled or clotted state.

As used herein, the term "gelled" means any state of elevated viscosity when compared to the initial state. This can be observed for example in the formation of fibrin from fibrinogen or in a finely dispersed system of at least one solid phase and at least one liquid phase, such as a colloid. Further, the term "gelled" includes all states of gelation known in the art.

The term "clotted" means, for example, a gel comprising fibrin and includes any kind of coagulation state known in the art.

According to the present invention, the viscosity of the composition included in the bag of the intervertebral disc implant depends on the application, i.e. the intervertebral disc disorder to be treated, and is adjusted within the common knowledge of a person skilled in the art. Preferably, the viscosity of the composition included in the bag of the intervertebral disc implant of the present invention ranges from about 100 mPas to about 1000 Pas.

In a preferred embodiment of the present invention, the composition further comprises autologous serum as defined above for the implantable system. In another preferred embodiment of the implant of the present invention, the composition further comprises cells selected from the group consisting of autologous stem or precursor cells, allogenic stem or precursor cells, autologous or allogenic periost cells, autologous or allogenic chondrocytes, autologous or allogenic nucleus pulposus cells, autologous or allogenic annulus fibrosus cells and combinations thereof. It is particularly preferable to use autologous nucleus pulposus cells, since autologous nucleus pulposus cells are suitable to form the basis for the regeneration of the nucleus pulposus in the damaged spinal disc. In particular, according to the present invention, it is shown that nucleus pulposus cells remain vital within the cell carrier medium and/or in combination with the fibrin/hyaluronic acid mixture, and express nucleus pulposus specific molecules.

In another preferred embodiment of the implant of the present invention, the composition further comprises ingredients selected from the group consisting of annulus fibrosus stimulating factors, annulus fibrosus specific matrix components, anti-inflammatory substances, nucleus pulposus stimulating factors, nucleus pulposus specific matrix components, and combinations thereof. This is advantageous in view of a further optimization of the regenerative processes within the damaged nucleus pulposus.

Further, it is preferred that the cell carrier medium is a bioresorbable polymer fleece comprising a material selected from the group consisting of collagens, hyaluronic acid, glycosaminoglycane, polylactic acid, polyglycolic acid, copolymers of polylactic acid and polyglycolic acid, and combinations thereof.

Moreover, the above composition may further include any other component suitable for e.g. augmenting, strengthening, supporting, repairing, rebuilding or healing a intervertebral disc, such as growth factors, chemotherapeutic or pharmacological agents, biologically active agents, hardening and/or adhesive compounds and mineral additives.

According to the present invention, an intervertebral disc implant is provided which is highly biocompatible, prevents the occurrence of re-herniation, provides a high initial biomechanical stability, and enables an adequate nutrient supply of new intervertebral disc tissue. In particular, the fibrin component obtained by the combination of fibrinogen and thrombin within the bag of the implant provides a gel-like component which is the basis for the substitute of the damaged nucleus pulposus. Further, it is shown that the combination of the cell carrier medium with hyaluronic acid leads to a partial restoration of the biomechanical stability. Thus, the enhanced range of motion of the intervertebral disc which is generally observed after a common surgical discectomy, can be significantly reduced by the intervertebral disc implant according to the present invention. This effect is a consequence of the high water-binding ability of hyaluronic acid which leads to a strong hydration and stabilization of the implant. Further, the use of the bag in the implant according to the present invention significantly reduces the possibility of a re-herniation, since the bag strengthens the annulus fibrosus from the inside and seals the annulus fibrosus defect.

The present invention further relates to a method for implanting the above implantable system into an intervertebral disc having an annulus fibrosus and/or nucleus pulposus defect, the method comprising the steps of introducing the bag comprising a cell carrier medium into an intervertebral disc space; filling components (a) to (c) of the multi-component system into the bag; and sealing the bag.

In this context, it is noted that the order of the above steps is not fixed but may be varied in any practical manner. For example, the step of sealing the bag may be carried out before or after introducing the bag into the intervertebral disc space and/or before or after filling components (a) to (c) of the multi-component system into the bag.

The step of filling may be carried out in any suitable manner. When the step of sealing the bag is performed before the step of filling the multi-component system into the bag, the bag may be filled for example by puncturing the sealed bag with the needle of a syringe and injecting the multi-component system into the bag through the puncture. When the bag has not yet been sealed, the multi-component system is preferably injected through the opening of the bag by means of a two-component syringe, wherein at least components (a) and (b) of the multi-component system are initially spacially separated from each other. By mixing the fibrinogen component (a) and the thrombin component (b), a solid gel-like material is formed *in situ* within the bag. Said gel-like material infiltrates the cell carrier medium and presses the bag against the inner walls of the intervertebral disc space. As a consequence, the bag strongly sticks to the inner walls of the intervertebral disc space, thus giving high stability to the implant within the intervertebral disc space. It is further possible to modify the step of filling the multi-component system into the bag in a way that pressure is applied when the multi-component system is injected into the bag. Such an applied pressure during the injection further enhances the stability of the implanted bag within the intervertebral disc space.

In a preferred embodiment, the multi-component system to be filled into the bag further comprises cells selected from the group consisting of autologous stem or precursor cells, allogenic stem or precursor cells, autologous or allogenic periost cells, autologous or allogenic chondrocytes, autologous or allogenic nucleus pulposus cells, autologous or allogenic annulus fibrosus cells and combinations thereof. In case autologous nucleus pulposus cells are included into the multi-component system to be filled into the bag, generally a two-stage procedure has to be applied. In particular, in a first step nucleus pulposus tissue is extracted from the patient's intervertebral disc. Then, after cultivating the nucleus pulposus cells *in vitro,* the autologous nucleus pulposus cells are implanted into the bag in a second step. According to the present invention, in order to minimize the problems associated with a second surgical intervention, different approaches are possible.

For example, in one preferred embodiment of the above method, in a first step, the bag is implanted into the intervertebral and components (a) to (c) of the multi-component system are filled into the bag. At the same time, nucleus pulposus cells are extracted from the patient's intervertebral disc. Further, a fine catheter is placed transannularly within in the bag and is conducted out percutane. In the second step, nucleus pulposus cells and preferably further substances and/or nutrients are introduced into the bag via said catheter. Thus, a second surgical intervention can be avoided.

In another preferred embodiment of the present invention, the second step is performed by means of a computer tomography supported puncture of the bag by which nucleus pulposus cells and preferably further substances and/or nutrients are introduced into the bag. Also this method avoids the need of a second surgical intervention.

Further, in another preferred embodiment of the present invention, the spinal disc prolapse itself is placed into the bag of the implant. In particular, during a surgical intervention the spinal disc prolapse is removed, and at the same time, the implant according to the present invention is implanted into the intervertebral disc space. Then, the removed spinal disc prolapse is introduced into the bag as a source of autologous nucleus pulposus cells. The advantage of this method is that no second surgical intervention is necessary. This procedure is particularly preferred in the case of a fresh spinal disc hernation, but not in the case of a strongly degenerated prolapse. Moreover, it is preferable to further add suited bioactive substances and nutrients into the bag at the same stage.

The step of sealing the bag may be performed by any means which are suitable for such purpose in a biologically acceptable manner. For example, the bag may be sealed with a suture e.g. in the form of a pursestring suture, and/or by means of suitable adhesives. Suitable adhesives are for example fibrin-based adhesives such as the commercially available TachoSil^{®}. In a preferred embodiment of the present invention, the bag is sealed by a pursestring suture. In case the bag comprises a neck-like opening, the surplus neck may be removed after suturing the bag. In another preferred embodiment of the present invention, the claimed method further comprises the step of suturing the bag with the annulus fibrosus. In particular, for additional security for preventing re-herniation, the bag may be fixed with the annulus fibrosus. This is also advantageous from the viewpoint of further fixing the implant within the intervertebral disc space.

In a further preferred embodiment of the present invention, the method for implanting the above implantable system into an intervertebral disc further comprises the step of puncturing the degenerated cartilaginous endplates of the vertebral bodies adjacent to the damaged intervertebral disc. Said puncturing may be performed by means of micro drillings and/or prickings. Such puncturing initiates the formation of new cartilaginous tissue which facilitates the supply with nutrients via diffusion of the intervertebral disc implant. In this context, also vasogenic factors may be applied.

In another preferred embodiment of the present invention, the claimed method further comprises the step of sealing the annulus fibrosus with a fibrin based sealant after sealing the bag. This further reduces the possibility of a re-hernation, since the annulus fibrosus is strengthened by the sealant and the annulus fibrosus defect is closed.

It is further preferred that the method of the present invention is performed minimal invasive and/or percutaneously. This is advantageous from the viewpoint of a reduced strain for the patient and a reduced risk compared to conventional techniques of surgical interventions.

Finally, the present invention relates to the use of the above implantable system for implanting into an intervertebral disc for the treatment of an annulus fibrosus and/or nucleus pulposus defect and/or for preventing an annulus fibrosus and/or nucleus pulposus defect.

As used herein, the term "annulus fibrosus and/or nucleus pulposus defect" means any state in which the annulus fibrosus and/or the nucleus pulposus is damaged. Typically, the annulus fibrosus and/or nucleus pulposus defect is associated with a spinal disc herniation, i.e. with a spinal disc protrusion and/or a spinal disc prolapse and/or intervertebral disc disease.

The present invention advantageously provides an implantable system, an intervertebral disc implant and the use of the above implantable system for implanting into an intervertebral disc. In particular, according to the present invention, it is surprisingly possible to provide a highly biocompatible implant for an intervertebral disc having high biomechanical stability. Further, it is possible to prevent the occurrence of re-herniation and to enable an adequate nutrient supply of new intervertebral disc tissue.

The figures show:
Figure 1 shows a two-component syringe containing the multi-component system according to the present invention.
Figure 2 shows the injection of the multi-component system into the biologically acceptable bag according to the present invention.
Figure 3 shows the infiltration of the biologically acceptable bag by the multi-component system according to the present invention.
Figure 4 shows the elastic filling of the biologically acceptable bag by sticking together of the external layers of the bag.
Figure 5 shows a fluorescence image of the bag according to the Example of the present invention (magnified 4 times) wherein green color indicates cells and red color indicates fleece fibers.
Figure 6 shows a fluorescence image of the bag according to the Example of the present invention (magnified 10 times) wherein green color indicates cells and red color indicates fleece fibers.

The present invention will now be further illustrated in the following Example.

### Example

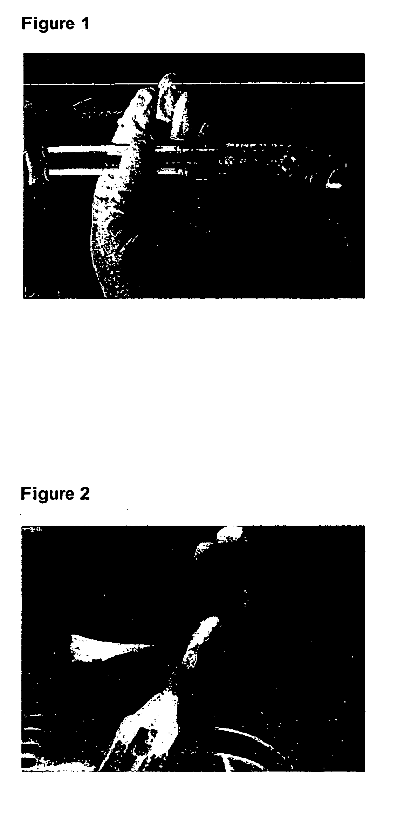

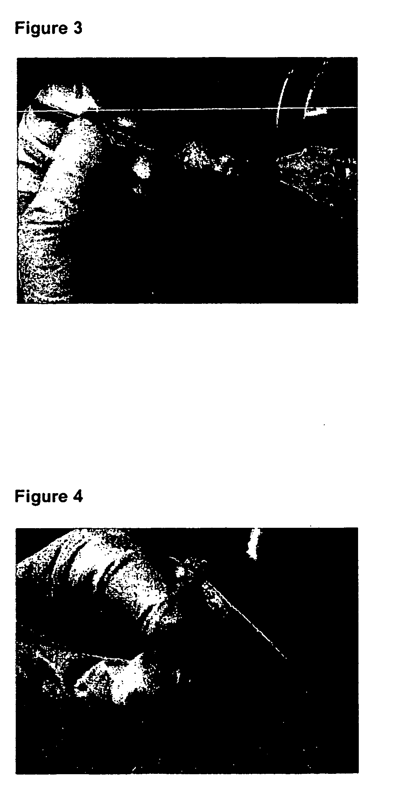

As an *in vitro* example, a bioresorbable polymer fleece is cut and sewed into the form of a bag having a neck-like opening. Additionally, a pursestring suture is prepared at the end of the neck-like opening. Then, a two-component syringe is filled with 850 µl of a thrombin-containing solution (first section of the two-component syringe), and 250 µl of a fibrinogen-containing solution, 300 µl of a hyaluronic acid-containing solution and 300 µl culture medium in which about 50 million nucleus pulposus cells have been dissolved before (second section of the two-component syringe). The filled syringe is shown in Figure 1. The nucleus pulposus cells are human nucleus pulposus cells which have been obtained from nucleus pulposus tissue of a discectomy wherein the cells have been isolated and expanded *in vitro.* Then, a small amount of the components is carefully injected into the bag via the neck-like opening. The injection of the components is shown in Figure 2. The fleece material is infiltrated (Figure 3), and by mixing the fibrinogen and the thrombin component, the fleece material sticks together within a short period of time.

Then, the remaining material of the two-component syringe is injected into the bag. Since the fleece material sticks together, the now injected material cannot leak out and the bag is properly filled. By pulling tight the pursestring suture around the needle of the syringe, a certain pressure can be applied during injection. Then, the pursestring suture is closed and the surplus neck of the bag is removed.

The resulting bag is elastically filled (Figure 4) and resists for example pressure applied by a pair of tweezers.

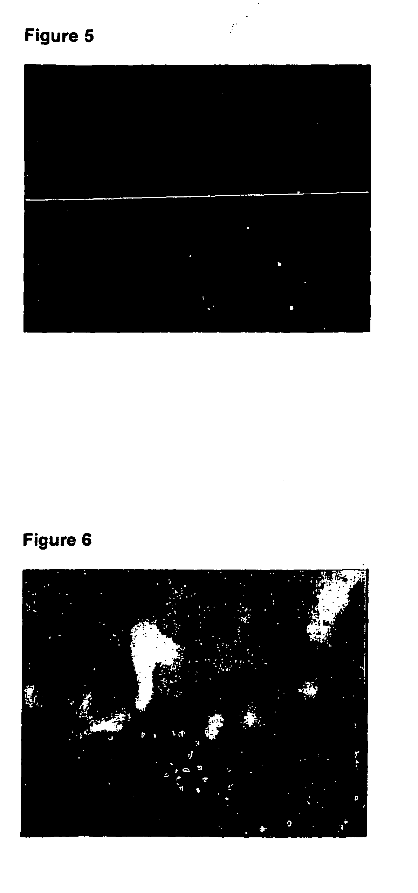

Then, the bag is kept in a defined culture medium for two weeks. Color changes indicate a lively metabolism. Finally, fluorescence microscopy is performed. Figures 5 and 6 show fluorescence images of the bag after two weeks demonstrating a high viability of the cells within the bag. In the Figures, green color indicates the cells and red color indicates the fleece fibers.

## Claims

1. An implantable system for an implant into an intervertebral disc, comprising
a biologically acceptable bag comprising a cell carrier medium; and
a multi-component system comprising:
component (a) containing fibrinogen;
component (b) containing thrombin; and
component (c) containing hyaluronic acid.

2. The implantable system according to claim 1, wherein components (a) to (c) are present in solution and at least component (a) is spacially separated from component (b).

3. The implantable system according to claim 1 or 2, wherein the multi-component system further comprises autologous serum.

4. The implantable system according to anyone of claims 1 to 3, wherein the multi-component system further comprises cells selected from the group consisting of autologous stem or precursor cells, allogenic stem or precursor cells, autologous or allogenic periost cells, autologous or allogenic chondrocytes, autologous or allogenic nucleus pulposus cells, autologous or allogenic annulus fibrosus cells and combinations thereof.

5. The implantable system according to anyone of claims 1 to 4, wherein the cell carrier medium is a bioresorbable polymer fleece comprising a material selected from the group consisting of collagens, hyaluronic acid, glycosaminoglycane, polylactic acid, polyglycolic acid, copolymers of polylactic acid and polyglycolic acid, and combinations thereof.

6. The implantable system according to anyone of claims 1 to 5, wherein the multi-component system further comprises ingredients selected from the group consisting of annulus fibrosus stimulating factors, annulus fibrosus specific matrix components, anti-inflammatory substances, nucleus pulposus stimulating factors, nucleus pulposus specific matrix components, and combinations thereof.

7. The implantable system according to anyone of claims 1 to 6, wherein the amount of fibrinogen in component (a) ranges from about 10 to about 200 mg/ml.

8. The implantable system according to anyone of claims 1 to 7, wherein the amount of thrombin in component (b) is at least about 1 IU/ml in the final clotted composition.

9. The implantable system according to anyone of claims 1 to 8, wherein the amount of hyaluronic acid in component (c) ranges from about 0.01 to about 5 mg/ml.

10. An intervertebral disc implant, comprising
a biologically acceptable bag comprising a cell carrier medium,
the bag comprising a composition comprising
component (a) containing fibrin;
component (b) containing thrombin; and
component (c) containing hyaluronic acid.

11. The implant according to claim 10, wherein the composition further comprises autologous serum.

12. The implant according to claim 10 or 11, wherein the composition further comprises cells selected from the group consisting of autologous stem or precursor cells, allogenic stem or precursor cells, autologous or allogenic periost cells, autologous or allogenic chondrocytes, autologous or allogenic nucleus pulposus cells, autologous or allogenic annulus fibrosus cells and combinations thereof.

13. The implant according to anyone of claims 10 to 12, wherein the cell carrier medium is a bioresorbable polymer fleece comprising a material selected from the group consisting of collagens, hyaluronic acid, glycosaminoglycane, polylactic acid, polyglycolic acid, copolymers of polylactic acid and polyglycolic acid, and combinations thereof.

14. The implant according to anyone of claims 10 to 13, wherein the composition further comprises ingredients selected from the group consisting of annulus fibrosus stimulating factors, annulus fibrosus specific matrix components, anti-inflammatory substances, nucleus pulposus stimulating factors, nucleus pulposus specific matrix components, and combinations thereof.

15. The implant according to anyone of claims 10 to 14, wherein the amount of fibrin in component (a) ranges from about 10 to about 200 mg/ml.

16. The implant according to anyone of claims 10 to 15, wherein the amount of thrombin in component (b) is at least about 1 IU/ml.

17. The implant according to anyone of claims 10 to 16, wherein the amount of hyaluronic acid in component (c) ranges from about 0.01 to about 5 mg/ml.

18. The implant according to anyone of claims 10 to 17, wherein the bag is sealed by a pursestring suture.

19. Use of the implantable system according to anyone of claims 1 to 9 for the preparation of an implant into an intervertebral disc for the treatment of an annulus fibrosus and/or nucleus pulposus defect and/or for preventing an annulus fibrosus and/or nucleus pulposus defect.

## Patentansprüche

1. Implantierbares System für ein Implantat in eine Bandscheibe, umfassend einen biologisch verträglichen Beutel, umfassend ein Zell-Trägermedium; und ein Mehrkomponentensystem, umfassend:
Komponente (a), enthaltend Fibrinogen;
Komponente (b), enthaltend Thrombin; und
Komponente (c), enthaltend Hyaluronsäure.

2. Implantierbares System nach Anspruch 1, wobei die Komponenten (a) bis (c) in Lösung vorliegen und wobei mindestens Komponente (a) von Komponente (b) räumlich getrennt ist.

3. Implantierbares System nach Anspruch 1 oder 2, wobei das Mehrkomponentensystem weiter autologes Serum umfasst.

4. Implantierbares System nach einem der Ansprüche 1 bis 3, wobei das Mehrkomponentensystem weiter Zellen umfasst, ausgewählt aus der Gruppe, bestehend aus autologen Stamm- oder Vorläuferzellen, allogenen Stamm- oder Vorläuferzellen, autologen oder allogenen Periostzellen, autologen oder allogenen Chondrocyten, autologen oder allogenen *Nucleus pulposus-*Zellen*,* autologen oder allogenen *Anulus fibrosus*-Zellen und Kombinationen davon.

5. Implantierbares System nach einem der Ansprüche 1 bis 4, wobei das Zell-Trägermedium ein bioresorbierbares Polymervlies ist, umfassend ein Material, ausgewählt aus der Gruppe, bestehend aus Kollagenen, Hyaluronsäure, Glycosaminoglycan, Polymilchsäure, Polyglykolsäure, Copolymeren von Polymilchsäure und Polyglykolsäure, und Kombinationen davon.

6. Implantierbares System nach einem der Ansprüche 1 bis 5, wobei das Mehrkomponentensystem weiter Bestandteile umfasst, ausgewählt aus der Gruppe, bestehend aus *Anulus fibrosus* stimulierenden Faktoren, *Anulus fibrosus-*typischen Matrixkomponenten, anti-inflammatorischen Substanzen, *Nucleus pulposus* stimulierenden Faktoren, *Nucleus pulposus*-typischen Matrixkomponenten und Kombinationen davon.

7. Implantierbares System nach einem der Ansprüche 1 bis 6, wobei die Menge an Fibrinogen in Komponente (a) etwa 10 bis etwa 200 mg/ml beträgt.

8. Implantierbares System nach einem der Ansprüche 1 bis 7, wobei die Menge an Thrombin in Komponente (b) mindestens etwa 1 IU/ml in der endgültigen geronnenen Zusammensetzung beträgt.

9. Implantierbares System nach einem der Ansprüche 1 bis 8, wobei die Menge an Hyaluronsäure in Komponente (c) etwa 0,01 bis etwa 5 mg/ml beträgt.

10. Bandscheibenimplantat, umfassend
einen biologisch verträglichen Beutel, umfassend ein Zell-Trägermedium, wobei der Beutel eine Zusammensetzung, umfassend
Komponente (a), enthaltend Fibrin;
Komponente (b), enthaltend Thrombin; und
Komponente (c), enthaltend Hyaluronsäure,
umfasst.

11. Implantat nach Anspruch 10, wobei die Zusammensetzung weiter autologes Serum umfasst.

12. Implantat nach Anspruch 10 oder 11, wobei die Zusammensetzung weiter Zellen umfasst, ausgewählt aus der Gruppe, bestehend aus autologen Stamm- oder Vorläuferzellen, allogenen Stamm- oder Vorläuferzellen, autologen oder allogenen Periostzellen, autologen oder allogenen Chondrocyten, autologen oder allogenen *Nucleus pulposus-*Zellen*,* autologen oder allogenen *Anulus fibrosus*-Zellen und Kombinationen davon.

13. Implantat nach einem der Ansprüche 10 bis 12, wobei das Zell-Trägermedium ein bioresorbierbares Polymervlies ist, umfassend ein Material, ausgewählt aus der Gruppe, bestehend aus Kollagenen, Hyaluronsäure, Glycosaminoglycan, Polymilchsäure, Polyglykolsäure, Copolymeren von Polymilchsäure und Polyglykolsäure, und Kombinationen davon.

14. Implantat nach einem der Ansprüche 10 bis 13, wobei die Zusammensetzung weiter Bestandteile umfasst, ausgewählt aus der Gruppe, bestehend aus *Anulus fibrosus* stimulierenden Faktoren, *Anulus fibrosus-typischen* Matrixkomponenten, anti-inflammatorischen Substanzen, *Nucleus pulposus* stimulierenden Faktoren, *Nucleus pulposus*-typische Matrixkomponenten und Kombinationen davon.

15. Implantat nach einem der Ansprüche 10 bis 14, wobei die Menge an Fibrin in Komponente (a) etwa 10 bis etwa 200 mg/ml beträgt.

16. Implantat nach einem der Ansprüche 10 bis 15, wobei die Menge an Thrombin in Komponente (b) mindestens etwa 1 IU/ml beträgt.

17. Implantat nach einem der Ansprüche 10 bis 16, wobei die Menge an Hyaluronsäure in Komponente (c) etwa 0,01 bis etwa 5 mg/ml beträgt.

18. Implantat nach einem der Ansprüche 10 bis 17, wobei der Beutel durch eine Tabaksbeutelnaht verschlossen ist.

19. Verwendung des implantierbaren Systems nach einem der Ansprüche 1 bis 9 für die Herstellung eines Implantats in eine Bandscheibe für die Behandlung eines *Anulus fibrosus-* und/oder *Nucleus pulposus-Defekts* und/oder für das Vorbeugen eines *Anulus fibrosus-* und/oder *Nucleus pulposus-Defekts.*

## Revendications

1. Système implantable, pour implantation dans un disque intervertébral, comprenant :
un sac biologiquement acceptable comprenant un milieu support de cellule, et
un système multi-composants comprenant :
un composant (a) contenant un fibrinogène,
un composant (b) contenant de la thrombine ; et
un composant (c) contenant de l'acide hyaluronique.

2. Système implantable selon la revendication 1, dans lequel les composants (a) à (c) sont présents en solution et au moins le composant (a) est séparé dans l'espace du composant (b).

3. Système implantable selon la revendication 1 ou 2, dans lequel le système multi-composants comprend en outre un sérum autologue.

4. Système implantable selon l'une quelconque des revendications 1 à 3, dans lequel le système multi-composants comprend en outre des cellules choisies dans le groupe constitué de cellules souches ou précurseurs autologues, de cellules souches ou précurseurs allogéniques, de cellules périostiques autologues ou allogéniques, de chondrocytes autologues ou allogéniques, de cellules de noyau pulpeux allogéniques ou autologues, de cellules d'annulus fibrosus autologues ou allogéniques, et des combinaisons correspondantes.

5. Système implantable selon l'une quelconque des revendications 1 à 4, dans lequel le milieu de support de cellule est une toison de polymère biorésorbable comprenant un matériau choisi dans le groupe constitué de collagènes, d'acide hyaluronique, de glycosaminoglycane, d'acide polylactique, d'acide polyglycolique, de copolymères d'acide polylactique et d'acide polyglycolique et des combinaisons correspondantes.

6. Système implantable selon l'une quelconque des revendications 1 à 5, dans lequel le système multi-composants comprend en outre des ingrédients choisis dans le groupe constitué de facteurs de stimulation d'annulus fibrosus, de composants de matrice spécifique d'annulus fibrosus, de substances anti-inflammatoires, de facteurs de stimulation de noyau pulpeux, de composants de matrice spécifiques de noyau pulpeux, et des combinaisons correspondantes.

7. Système implantable selon l'une quelconque des revendications 1 à 6, dans lequel la quantité de fibrinogène dans le composant (a) est comprise entre environ 10 et environ 200 mg/ml.

8. Système implantable selon l'une quelconque des revendications 1 à 7, dans lequel la quantité de thrombine dans le composant (b) est d'au moins environ 1 IU/ml dans la composition coagulée finale.

9. Système implantable selon l'une quelconque des revendications 1 à 8, dans lequel la quantité d'acide hyaluronique dans le composant (c) est comprise entre environ 0,01 et environ 5 mg/ml.

10. Implant de disque intervertébral, comprenant :
un sac biologiquement acceptable comprenant un milieu support de cellule,
le sac comprenant une composition comprenant :
un composant (a) contenant de la fibrine,
un composant (b) contenant de la thrombine ; et
un composant (c) contenant de l'acide hyaluronique.

11. Implant selon la revendication 10, dans lequel la composition comprend en outre un sérum autologue.

12. Implant selon la revendication 10 ou 11, dans lequel la composition comprend en outre des cellules choisies dans le groupe constitué de cellules souches ou précurseurs autologues, de cellules souches ou précurseurs allogéniques, de cellules périostiques autologues ou allogéniques, de chondrocytes autologues ou allogéniques, de cellules de noyaux pulpeux allogéniques ou autologues, de cellules d'annulus fibrosus autologues ou allogéniques, et des combinaisons correspondantes.

13. Implant selon l'une quelconque des revendications 10 à 12, dans lequel le milieu support de cellule est une toison de polymère biorésorbable comprenant un matériau choisi dans le groupe constitué de collagènes, d'acide hyaluronique, de glycosaminoglycane, d'acide polylactique, d'acide polyglycolique, de copolymères d'acide polylactique et d'acide polyglycolique et des combinaisons correspondantes.

14. Implant selon l'une quelconque des revendications 10 à 13, dans lequel la composition comprend en outre des ingrédients choisis dans le groupe constitué de facteurs de stimulation d'annulus fibrosus, de composants de matrice spécifique d'annulus fibrosus, de substances anti-inflammatoires, de facteurs de stimulation de noyau pulpeux, de composants de matrice spécifiques de noyau pulpeux, et des combinaisons correspondantes.

15. Implant selon l'une quelconque des revendications 10 à 14, dans lequel la quantité de fibrine dans le composant (a) est comprise entre environ 10 et environ 200 mg/ml.

16. Implant selon l'une quelconque des revendications 10 à 15, dans lequel la quantité de thrombine dans le composant (b) est d'au moins environ 1 IU/ml.

17. Implant selon l'une quelconque des revendications 10 à 16, dans lequel la quantité d'acide hyaluronique dans le composant (c) est comprise entre environ 0,01 et environ 5 mg/ml.

18. Implant selon l'une quelconque des revendications 10 à 17, dans lequel le sac est scellé par une suture en bourse.

19. Utilisation du système implantable selon l'une quelconque des revendications 1 à 9, pour la préparation d'un implant dans un disque intervertébral pour le traitement d'un défaut de l'annulus fibrosus et/ou du noyau pulpeux et/ou pour la prévention d'un défaut de l'annulus fibrosus et/ou du noyau pulpeux.
